Europäisches Patentamt

European Patent Office

Office européen des brevets

(19)

(11) Publication number: **0 055 945**
A2

(12)

# EUROPEAN PATENT APPLICATION

(21) Application number: **81306193.4**

(22) Date of filing: **31.12.81**

(51) Int. Cl.³: **C 12 N 15/00**
C 12 P 21/02, C 07 C 103/52

(30) Priority: **02.01.81 US 222044**

(43) Date of publication of application:
**14.07.82 Bulletin 82/28**

(84) Designated Contracting States:
**AT BE CH DE FR GB IT LI LU NL SE**

(71) Applicant: GENENTECH, INC.
460 Point San Bruno Boulevard
So. San Francisco California 94080(US)

(72) Inventor: Goeddel, David V.
1449 Benito
Burlingame California 94010(US)

(72) Inventor: Kleid, Dennis G.
724 Costa Rica Avenue
San Mateo California 94402(US)

(72) Inventor: Itakura, Keiichi
1450 East Duarte Road
Duarte California 91010(US)

(74) Representative: Armitage, Ian Michael et al,
MEWBURN ELLIS & CO. 2/3 Cursitor Street
London EC4A 1BQ(GB)

(54) Human proinsulin and analogs thereof and method of preparation by microbial polypeptide expression and conversion thereof to human insulin.

(57) Microbial expression of a chimeric gene is used to produce a polypeptide comprising the amino acid sequence of human proinsulin, or an analog thereof differing in the "C" chain portion. A polypeptide so produced contains a sequence of additional amino acid units sufficient in number to protect it from bacterial proteases, and has a cleavage site e.g. a methionine residue adjacent the sequence of amino acid units corresponding to the proinsulin or proinsulin analog. Cleavage at this site (e.g. by CNBr) generates proinsulin (or the analog) which is treated *in vitro* to form the disulfide bonds between the "A" and "B" chain proteins characteristic of human insulin. The "C" chain portion is then excised enzymatically to yield human insulin useful e.g. in the treatment of diabetes.

The chimeric gene may be synthesised from oligonucleotides and inserted into a plasmid which is used to transform a host cell, e.g. *E. coli.*

./...

```
            1                                    10                                    20
    met phe val asn gln his leu cys gly ser his leu val glu ala leu tyr leu val cys gly
   N1              N2                   N3              N4              B1              B2
AATTC ATG TTC GTC AAT CAG CAC CTT TGT GGT TCT CAC CTC GTT GAA GCT TTG TAC CTT GTT TGC GGT
  G TAC AAG CAG TTA GTC GTG GAA ACA CCA AGA GTG GAG CAA CTT CGA AAC ATG GAA CAA ACG CCA
       N5                  N6                   N7              N8              B4


                                         30                                    40
glu arg gly phe phe tyr thr pro lys thr arg arg glu ala glu asp leu gln val gly gln val glu
    B3              B4                   B5
GAA CGT GGT TTC TTC TAC ACT CCT AAG ACT CGC CGG GAG GCA GAG GAC CTG CAG CTG GGG CAG GTG GAG
CTT GCA CCA AAG AAG ATG TGA GGA TTC TGA GCG GGC CTC CGT CTC CTG GAC GTC CAC CCC GTC CAC CTC
   B7                  B8                   B9              B10


                                    50                                    60
leu gly gly gly pro gly ala gly ser leu gln pro leu ala leu glu gly ser leu gln lys arg gly
CTG GGC GGC GGC CCT GGT GCA GGC AGC CTG CAG CCC TTG GCC CTG GAG GGC TCC CTG CAG AAG CGT GGC
GAC CCG CCG CCG GGA CCA CGT CCG TCG GAC GTC GGG AAC CGG GAC CTC CCC AGG GAC GTC TTC GCA CCG


                              70                                    80
ile val glu gln cys cys thr ser ile cys ser leu tyr gln leu glu asn tyr cys asn
ATT GTG GAA CAA TGC TGT ACC AGC ATC TGC TCC CTC TAC CAG CTG GAG AAC TAC TGC AAC TAG ACGCAGC
TAA CAC CTT GTT ACG ACA TGG TCG TAG ACG AGG GAG ATG GTC GAC CTC TTG ATG ACG TTG ATC TGCGTCG


CCGCAGGCAGCCCCACACCCGCCGCCTCCTGCACCGAGAGAGATGGAATAAAGCCCTTGAACCAGC-polyA-CCG
GGCGTCCGTCGGGGTGTGGGCGGCCGGAGGACGTGGCTCTCTCTACCTTATTTCGGGAACTTGGTCG-polyT-GGCCTAG
```

PROINSULIN SYNTHETIC GENE

Amino acids 1-30 are the B chain; 31-65 are the C chain; and 66-86 are the A chain.

FIG.I.

HUMAN PROINSULIN AND ANALOGS THEREOF
AND METHOD OF PREPARATION BY MICROBIAL
POLYPEPTIDE EXPRESSION AND
CONVERSION THEREOF TO HUMAN INSULIN

## Related Applications

This application is related to and incorporates by
reference the disclosures of European Patent Application
Publications Nos. 0001930 (A.N. 78300597.8) and 0036776
(A.N. 81301227.5).

## Field of the Invention

This invention relates to microbial expression of
polypeptides. In one aspect, it relates to the preparation of
genes for the microbially expressible production of
intermediates useful in the preparation of human insulin. In
another aspect, it relates to the preparation of human
proinsulin or analogs thereof differing from human proinsulin
in the "C" chain portion. In yet another aspect, it relates to
the preparation of human insulin from the prepared human
proinsulin or an analog thereof.

## Background of the Invention

Diabetes, the human condition characterized by a failure of the pancreas to generate the polypeptide hormone insulin in sufficient quantities, in severe cases at least, is currently treated by injection of insulin derived from the pancreas of slaughtered animals. Bovine and porcine insulin, in particular, are used for this purpose.

The use of insulin derived from animals is unsatisfactory from at least two standpoints. In the first place, the extraction of insulin from the pancreas of slaughtered animals is a complex process that requires large quantities of the organs. Secondly, and more importantly from the diabetic's point of view, the insulin derived from animal sources is not chemically identical to human insulin, differing in the sequence of peptide units. Furthermore, it sometimes contains non-homologous animal hormones, such as the corresponding proinsulin, albeit in small quantities. As a result, the response of patients treated with animal derived insulin is not as satisfactory as desired. For example, an immune response to animal insulin is believed to be a source of chronic complications in certain treatments of diabetes.

Accordingly, there has gone unfilled a long felt need to have a source of insulin identical chemically to human insulin, uncontaminated by other biologically active impurities, in amounts sufficient to permit diabetics to be treated economically. Complicating this task is the complex chemical structure of human insulin. Structurally it has two polypeptide chains referred to as the "A" and "B" chains bound to each other by disulfide bonds. The A chain, some 21 amino acid units in length, is bound (crosslinked) to the B chain, a chain of 30 amino acids, through disulfide bonds between units of the amino acid cysteine in each chain.

0055945

-3-

To be maximally effective in humans, the amino acid units of insulin must be precisely ordered to correspond to that produced in vivo. However, the complexity of the molecule is such that conventional methods of chemical synthesis are unsuited to its preparation, on a commercial scale at least.

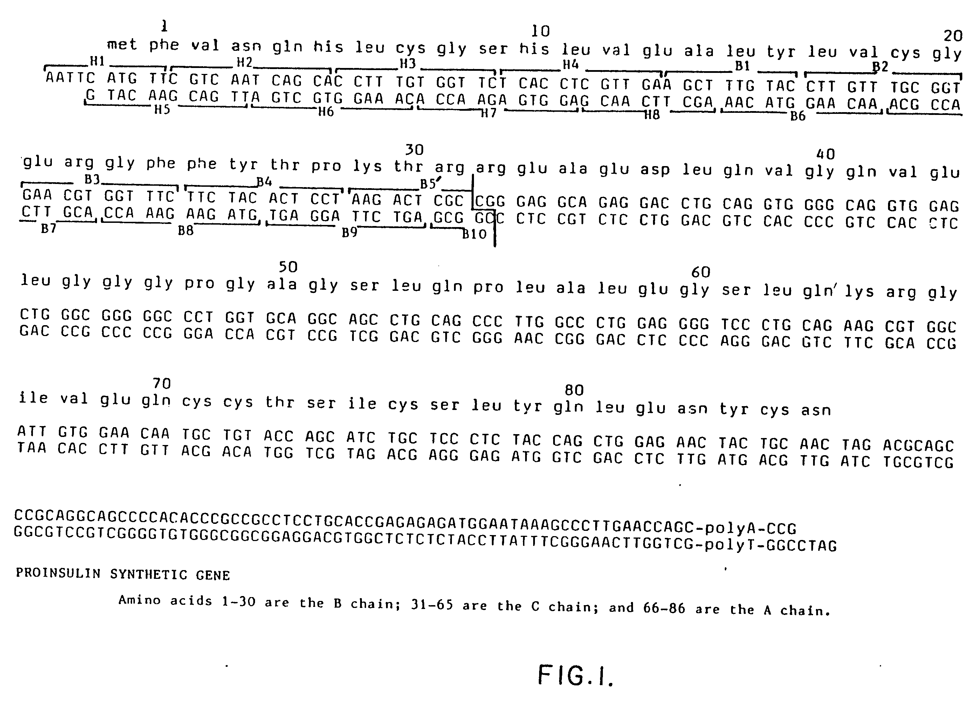

Insulin is produced in vivo in the pancreas in the form of preproinsulin. Preproinsulin is a polypeptide comprising the 21 units of the A chain, the 30 units of the B chain, a bridging or connecting chain of 35 units referred to as the C chain and a 24 amino acid "presequence" (Met Ala Leu Trp Met Arg Leu Leu Pro Leu Leu Ala Leu Leu Ala Leu Trp Gly Pro Asp Pro Ala Ala Ala) attached to the N-terminal phenylalanine amino acid beginning the B chain. Proinsulin, lacking the presequence is shown in Figure 1 with a methionine amino acid in place of the presequence. This presequence may participate in secretion from the cells in which it is produced. As the preproinsulin is excreted from the islet cells on the pancreas, the presequence is excised to leave the proinsulin chain. This chain folds to a structure in which three disulfide bonds are formed, two of which are between the A and B chain segments of the proinsulin. The connecting C chain is then excised proteolytically to leave a residue which is insulin, consisting of the A and B chains bound together by the disulfide bonds.

This application describes a method for obtaining human insulin and human proinsulin and analogs thereof which differ from human proinsulin in the sequence of amino acids making up the C chain. The method utilizes the burgeoning recombinant DNA technology. The following discussion of elements of the technology provide background to the detailed description of the invention.

With the advent of recombinant DNA technology, the controlled bacterial production of useful polypeptides has become possible. Already in hand are bacteria modified by this

-4-

technology to permit the production of such polypeptide products as somatostatin (K. Itakura et al., Science 198, 1056 (1977), the (component) A and B chains of human insulin (D.V. Goeddel et al., Proc. Nat'l. Acad. Sci. USA 76, 106 (1979)) and human growth hormone (D.V. Goeddel et al., Nature 281, 544 (1979)). Such is the power of the technology that virtually any useful polypeptide may be bacterially produced, putting within reach the controlled manufacture of hormones, enzymes, antibodies, and vaccines useful against a wide variety of diseases. The cited materials, which describe in greater detail the representative examples referred to above, are incorporated herein by reference, as are other publications referred to infra, to illuminate the background of the invention.

The work horse of recombinant DNA technology is the plasmid, an extra-chromosomal loop of double-stranded DNA found in bacteria, oftentimes in multiple copies per bacterial cell. Included in the information encoded in the plasmid DNA is that required to reproduce the plasmid in daughter cells (i.e., a "replicon") and ordinarily, one or more selection characteristics, such as resistance to antibiotics, which permit clones of the host cell containing the plasmid of interest to be recognized and preferentially grown in selective media. The utility of plasmids, which can be recovered and isolated from the host microorganism, lies in the fact that they can be specifically cleaved by one or another restriction endonuclease or "restriction enzyme", each of which recognizes a different site on the plasmidic DNA. Thereafter heterologou genes or gene fragments may be inserted into the plasmid by endwise joining at the cleavage site or at reconstructed ends adjacent the cleavage site.

As used herein, the term "heterologous" refers to a gene not ordinarily found in, or a polypeptide sequence ordinarily not produced by, the host microorganism whereas the term "homologous" refers to a gene or polypeptide which is produced in the host microorganism, such as E. coli. DNA recombination is performed outside the microorganisms but the resulting "recombinant" plasmid can be introduced into microorganisms by a process known as transformation and large quantities of the heterologous gene-containing recombinant plasmid obtained by growing the transformant. Moreover, where the gene is properly inserted with reference to portions of the plasmid which govern the transcription and translation of the encoded DNA message, the resulting plasmid or "expression vehicle", when incorporated into the host microorganism, directs the production of the polypeptide sequence for which the inserted gene codes, a process referred to as expression.

Expression is initiated in a region known as the promoter which is recognized by and bound by RNA polymerase. In some cases, as in the trp operon discussed infra, promoter regions are overlapped by "operator" regions to form a combined promoter-operator. Operators are DNA sequences which are recognized by so-called repressor proteins which serve to regulate the frequency of transcription initiation at a particular promoter. The polymerase travels along the DNA, transcribing the information contained in the coding strand from its 5' to 3' end into messenger RNA which is in turn translated into a polypeptide having the amino acid sequence for which the DNA codes. Each amino acid is encoded by a unique nucleotide triplet or "codon" within what may for present purposes be referred to as the "structural gene", i.e. that part which encodes the amino acid sequence of the expressed product. After binding to the promoter, the RNA polymerase first transcribes nucleotides encoding a ribosome binding site, then a translation initiation or "start" signal (ordinarily ATG, which in the resulting messenger RNA becomes AUG), then the nucleotide codons within the structural gene itself. So-called stop codons are transcribed at the end of the structural gene whereafter the polymerase may form an additional sequence of messenger RNA which, because of the presence of the stop signal, will remain untranslated by the ribosomes. Ribosomes bind to the binding site provided on the messenger RNA, in bacteria ordinarily as the mRNA is being formed, and themselves produce the encoded polypeptide, beginning at the translation start signal and ending at the previously mentioned stop signal. The desired product is produced if the sequences encoding the ribosome binding site are positioned properly with respect to the AUG initiator codon and if all remaining codons follow the initiator codon in phase. The resulting product may be obtained by lysing the host cell and recovering the product by appropriate purification from other microorganism protein.

Polypeptides expressed through the use of recombinant DNA technology may be entirely heterologous, as in the case of the direct expression of human growth hormone, or alternatively may comprise a heterologous polypeptide and, fused thereto, at least a portion of the amino acid sequence of a homologous peptide, as in the case of the production of intermediates for somatostatin and the components of human insulin. In the latter cases, for example, the fused homologous polypeptide comprised a portion of the amino acid sequence for beta galactosidase. In those cases, the intended bioactive product is bioinactivated by the fused, homologous polypeptide until the latter is cleaved away in an extracellular environment. Fusion proteins like those just mentioned can be designed so as to permit highly specific cleavage of the precursor protein from the intended product, as by the action of cyanogen bromide

-7-

on methionine, or alternatively by enzymatic cleavage. See, eg., G.B. Patent Publication No. 2 007 676 A.

Human insulin has hitherto been obtained employing techniques of recombinant DNA technology. The process used a synthetic gene for the A chain which is expressed in E. coli and a separate synthetic gene for the B chain which is expressed in another E. coli. D.V. Goeddel et al., Proc. Nat. Acad. Sci., USA, 76, 106 (1979). The two chains are obtained as chimeric polypeptides (proteins) comprising the desired sequence of amino acids (either the A or B chain sequence) bound to another section of carrier polypeptide designed to protect the desired sequence from proteases in the E. coli. The chimeric proteins have a selective cleavage site adjacent the desired polypeptide sequence of the A or B chain which permits separation of the desired sequence from the carrier polypeptide. Isolation of the two sequences is followed by the formation in vitro of the disulfide bonds.

This process is necessarily complicated by the fact that two distinct genetically modified bacterial strains must be obtained and maintained. Further, the prior process requires separate isolation of the A and B chain and the crosslinking of the two chains by means of the formation of the disulfide bonds without the aid in orientation provided by an intact C chain.

The present application describes a process for the construction of a single gene to express, in a single microorganism, a chimeric protein which includes a complete human proinsulin polypeptide or an analog thereof differing from human insulin in only the amino acid sequence of the C chain. Human insulin can be cleanly excised from these polypeptides after in vitro formation of the disulfide crosslinks between the A and B chains.

By this process, proinsulin, and analogs thereof, can be directly obtained in substantially pure form and free of biologically active impurities. Similarly, the proinsulins can be effectively processed to obtain insulin chemically identical to human insulin similarly free of biologically active impurities thus promising a more effective treatment of human diabetes than possible using animal derived insulin.

## Summary of the Invention

The present invention provides a method for obtaining human insulin by means of a chimeric polypeptide comprising the polypeptide sequence of human proinsulin, or an analog thereof differing from the polypeptide sequence of human proinsulin in the sequence of amino acids comprising the C chain, fused to additional protein or protein fragment, there being a selective cleavage site which permits cleavage of the proinsulin or its analog from the additional protein or fragment. The cleaved proinsulin product may then be caused to orient by formation of the characteristic insulin A and B chain disulfide crosslinks and the crosslinked insulin precursor may then be excised from the "C" chain carrier.

The "C" chain (or, hereinafter, bridging chain) of amino acid units of the analog proinsulins made according to the present invention may comprise as few as 2 amino acid units. The identity and sequence of amino acid units intermediate the ends of the bridging chains in analogs of human proinsulin are not particularly significant. However, the end units thereof must be units which permit facile excision of the bridging chain from the A and B chains of human insulin. Preferably, excision occurs after the proinsulin molecule has been cleaved from the addition protein of the chimeric polypeptide and, most preferably, after the thus cleaved proinsulin molecule has been folded and the disulfide links between the A and B chains characteristic of human insulin have been formed.
Preferably, the bridging chain has sites which permit its
excision by enzymatic means. Preferred for this purpose
are Arg-Arg and Lys-Arg units on the bridging chain which are
adjacent the terminal -COOH end of the B chain and terminal
$-NH_2$ end of the A chain, respectively, as is found in human
proinsulin itself. Also preferred are two Arg-Arg units
between the B and A chains.

The chimeric proteins of the present invention are obtained
by expression of a heterologous structural gene for the
proinsulin or analog in a recombinant microbial cloning vehicle
in which the gene is in reading phase with a DNA sequence
coding for an addition protein portion of the chimeric protein
and the cleavage site. In preferred embodiments of the
invention, the cleavage site is methionine at the N-terminal of
the proinsulin which permits cleavage using cyanogen bromide.

The additional protein can vary but the preferred
additional protein is methionine amino acid or the presequence
of preproinsulin or a portion thereof or β-galactosidase
or a substantial portion thereof or a portion of the amino acid
sequence encoded by a fragment of the trp leader polypeptide
gene fused to a portion of the trp E polypeptide gene or the
trp D polypeptide. The added, ultimately superfluous portion
of the chimeric protein is selected to provide protection from
bacterial proteases which might otherwise digest the proinsulin.

The preferred recombinant microbial cloning vehicle is a
modified, preferably bacterial, plasmid containing the
structural gene in a reading phase with the DNA sequence which
codes for the added, superfluous portion of the chimeric
polypeptide and the selective cleavage site.

The manner in which these and other objects and advantages
of the invention are achieved will be apparent to those skilled
in the art after consideration of the following description of the preferred embodiments and the illustrations of Figs. 1-6.

## Brief Description of the Drawings

Figure 1 shows the nucleotide sequence of a gene for human proinsulin.

Figure 2 illustrates a scheme for obtaining a plasmid containing a fragment of the gene of Figure 1 which was derived using reverse transcription from mRNA.

Figure 3 illustrates a scheme for obtaining a plasmid containing the gene of Figure 1 su: ble for transformation into, e.g., E. coli for expression of human proinsulin.

Figure 4 illustrates the analysis by HPLC chromatography of human proinsulin obtained by expression from an, e.g., E. coli transformant containing the plasmid derived from the scheme of Figure 3.

Figure 5 illustrates segments of a gene for expression of an analog of human proinsulin differing from human proinsulin in the amino acid sequence of the C, bridging chain.

Figure 6 illustrates a scheme for assembling a plasmid containing a gene for transformation into, e.g., E. coli for expression of an analog of human proinsulin.

## Detailed Description

A.  Preparation of Human Proinsulin

1.  Preparation of Synthetic Gene Coding For The 32 N-Terminal Amino Acids of Proinsulin

a.  Oligonucleotide Synthesis

A series of 18 oligonucleotides, short nucleotide chains 10-12 units in length shown in Table 1, were prepared as a first step to the construction of a gene coding for the first 32 amino acids of proinsulin, the amino acid sequence of which is shown in Fig. 1 as a part of the nucleotide sequence of the entire gene ultimately constructed for use in the present

-11-

invention for the expression of human proinsulin. The
individual nucleotides in the gene are identified by the
letters A, T, C or G representing the bases adenine, thymine,
cytosine or guanine which distinguish one nucleotide from
another.

TABLE 1

Oligonucleotides:

| | |
|---|---|
| H1 | AATTCATGTT |
| H2 | CGTCAATCAGCA |
| H3 | CCTTTGTGGTTC |
| H4 | TCACCTCGTTGA |
| H5 | TTGACGAACATG |
| H6 | CAAAGGTGCTGA |
| H7 | AGGTGAGAACCA |
| H8 | AGCTTCAACG |
| B1 | AGCTTTGTAC |
| B2 | CTTGTTTGCGGT |
| B3 | GAACGTGGTTTC |
| B4 | TTCTACACTCCT |
| B5' | AAGACTCGCC |
| B6 | AACAAGGTACAA |
| B7 | ACGTTCACCGCA |
| B8 | GTAGAAGAAACC |
| B9 | AGTCTTAGGAGT |
| B10' | GATCCGGCG |

The synthetic nucleotides are shown between brackets in Fig. 1. These oligonucleotides were synthesized by the triester method: R. Crea et al., Proc. Nat. Acad. Sci., USA, 75, 5765 (1978), K. Itakura et al., J. Biol. Chem., 250, 4592 (1975) and K. Itakura et al., J. Am. Chem. Soc., 97, 7327 (1975). Some of these are oligonucleotides which were used in a gene coding for the B chain of human insulin previously described by Crea et al., Proc. Nat. Acad. Sci., USA, 75, 5765 (1978) and Goeddel et al. Proc. Nat. Acad. Sci., USA, 76, 106 (1979). The nucleotide sequences of two synthetic nucleotides (B5' and B10'), were synthesized for this project; the others were prepared according to Crea et al. (supra.) The two new oligonucleotides, also prepared according to Crea et al., incorporate restriction enzyme recognition sites for HpaII and terminal BamHI, the latter used for cloning. The other end of the gene contains a sticky end of an EcoRI site for cloning purposes.

b. Joining of Synthetic Oligonucleotides

The eight oligonucleotides H1-H8 were used previously to construct the left half of the B chain gene. This was used in this process and is described by Goeddel et al., Proc. Natl. Acad. Sci. USA 76, 106 (1979). It contains the codons for the 1-13 amino acids of the B chain gene and a methionine unit at the N-terminal, used later to cleave the proinsulin from bacterially expressed chimeric protein using cyanogen bromide (CNBr).

The right half of the B chain gene was obtained from the oligonucleotides $B_1$, $B_2$, $B_3$, $B_4$, $B_5'$, $B_6$, $B_7$, $B_8$, $B_9$ and $B_{10}'$, by ligation using $T_4$ ligase and a technique described by Goeddel et al. (supra). The gene fragment produced codes for the 14-30 amino acid units of the B chain and the first unit, Arg, of the bridging chain.

Incorporated into the gene sequence is an HpaII restriction enzyme site in the same reading frame and location as an HpaII site in the human insulin gene. After purification of the ligated gene fragment by polyacrylamide gel electrophoresis, and elution of the largest DNA band, the fragment was inserted into the plasmid pBR322 that had been cleaved with restriction endonucleases HindIII and BamHI, thereby utilizing the HindIII and BamHI sites on the synthetic gene fragment. The DNA was inserted into E. coli 294 (ATCC No. 31445) by transformation. One plasmid, pB3' recovered from an ampicillin resistant, tetracycline sensitive clone was found to possess the desired nucleotide sequence according to the method of A.M. Maxam et al. Proc. Natl. Acad. Sci USA 74, 560 (1977).

From the two plasmids pBH 1 and pB3', two DNA fragments were recovered, a 46 base pair EcoRi to HindIII fragment from pBH1, and a 58 base pair HindIII to BamHI fragment from pB3'.

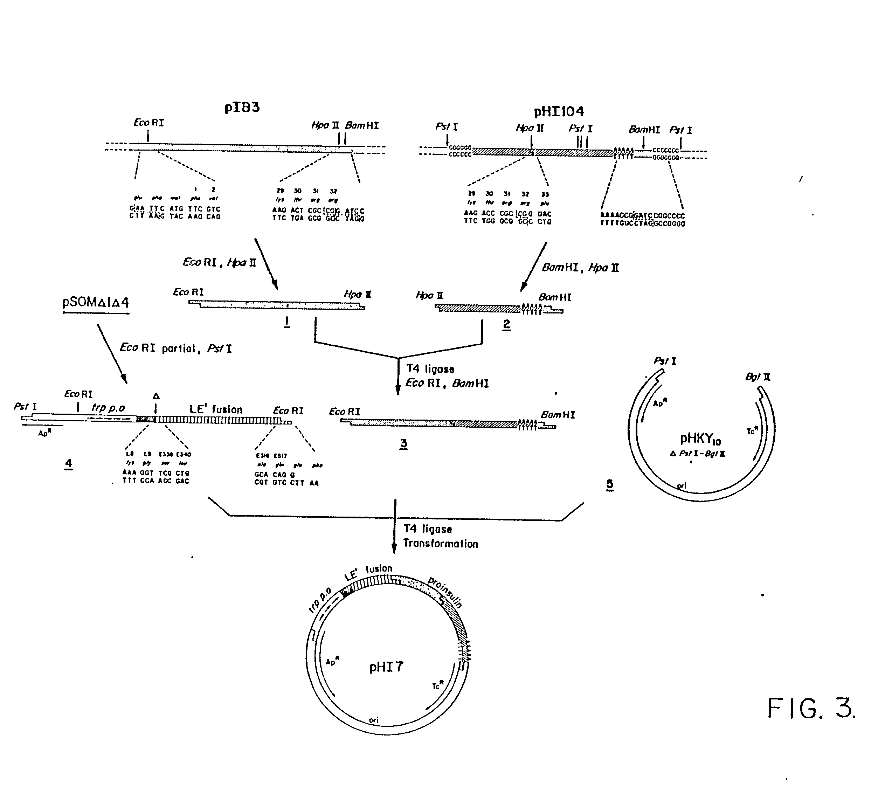

The two fragments were ligated together to produce a fragment having an EcoRI site and a BamHI site. This fragment was inserted in plasmid pBR322 which had been treated with EcoRI and BamHI restriction endonucleases using the method described in Goeddel et al. Proc. Nat. Acad. Sci., USA, 75, 106 (1979) and cloned in E. coli K-12 strain 294 (ATCC No. 31446) to provide the plasmid pIB3. After cloning, the plasmid pIB3 was cleaved with EcoRI and HpaII restriction endonucleases to recover the synthetic gene fragment (Fragment 1, Figure 3) containing the codons for the N-terminal proinsulin amino acids preceded by a methionine codon as shown in Figs. 1 and 3. The synthetic gene was isolated by polyacrylamide gel electrophoresis.

2. Isolation of A cDNA Gene Coding For the 55 C-Terminal Amino Acids of Human Proinsulin

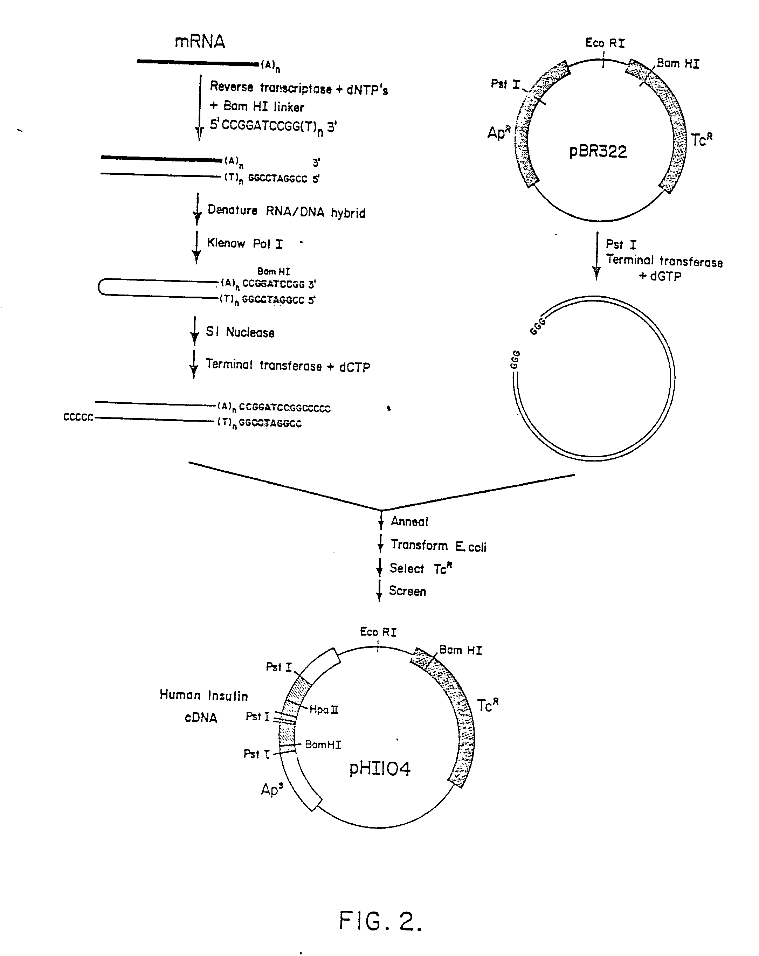

The scheme for obtaining the cDNA gene is schematically shown in Fig. 2.

A decanucleotide was synthesized containing the recognition sequence for BamHI endonucleases to which was added a 3' polythymidylic acid tract of approximately 20 residues. Its sequence is pCCGGATCCGGTT$_{18}$T. This oligonucleotide was used to prime AMV reverse transcriptase for cDNA synthesis.

The primer was prepared using terminal deoxynucleotidyl transferase (Enzo Biochem, 200 units) with one μmole of the BamHI decanucleotide in a reaction volume of 0.6 ml containing $1.5 \times 10^{-4}$ TTP. The reaction was conducted at 37°C for one hour in a buffer system described by A. Chang et al. Nature, 275, 617 (1978).

Human insulinoma polyA tissue (2.5 μg) provided by the Institute fuer Diabetesforschung, Muenchen, West Germany (Dr. Wolfgang Kemmler) containing mRNA isolated by the process of Ullrich et al, Science, 196, 1313 (1977) was converted to double stranded cDNA by a procedure according to Wickens et al. J. Biol. Chem, 253, 2483 (1978). Thus, 80 μl containing 15 mM Tris/HCl (pH 8.3 at 42°C), 21 mM KCl, 8mM MgCl$_2$, 30 mM ß-mercaptoethanol, 2 mM of the primer dCCGGATCCGGTT$_{18}$T, and 1 mM dNTPs was preincubated at 0°C. Then 40 units of AMV reverse transcriptase were added and the mixture incubated for 15 minutes at 42°C.

The complementary cDNA strand was synthesized in a volume of 150 μl containing 25 mM Tris/HCl (pH 8.3), 35 mM KCl, 4 mM MgCl$_2$, 15 mM ß-mercaptoethanol and 9 units of DNA polymerase I (Klenow fragment). The mixture was incubated at 15°C for 90 minutes followed by 15 hours at 4°C. S1 nuclease digestion was then performed for 2 hours at 37°C using 1000 units of S1 nuclease (Miles Laboratories) as described by Wickens et al. supra. The double stranded cDNA (0.37 μg) was subjected to electrophoresis on an 8 percent polyacrylamide gel. DNA fragments larger than 500 base pairs were eluted. Oligodeoxycytidylic acid residues were added to the 3' ends of the fragments using terminal deoxynucleotidyl transferase by the procedure of J.V. Maizel Jr., Meth. Virol., 5, 180 (1971). The dC tailed cDNA fragments were annealed to pBR322 that had been cleaved with the restriction endonuclease PstI and tailed with deoxyguanidylic acid using terminal deoxynucleotidyl transferase. The resulting plasmids were transformed into E. coli K-12 strain 294 and cloned. Colonies resistant to tetracycline but sensitive to ampicillin were isolated and screened for plasmids having three sites cleavable by the restriction endonuclease PstI indicative of the presence of the gene for insulin. Sures et al. Science, 208, 57 (1980).

One plasmid, pHI104, containing a 600 base pair insert and giving the anticipated PstI restriction pattern was determined to contain a site cleavable by BamHI between the 3' polyA and the polyGC introduced during the cDNA preparation. Some of the nucleotide sequence of the insert is shown in Fig. 1. This sequence differs slightly from that previously reported by I. Sures et al. Science, 208, 57 (1980) and G. Bell, et al. Nature, 282, 525 (1979), having an AT base pair where underlined rather than a CG pair, because the mRNA used was from tissue isolated from a different individual. The resistance to antibiotics conferred on a bacterium by this plasmid is indicated by the marker $Ap^S$ for ampicillin sensitivity and $Tc^r$ for tetracycline resistance.

3. Assembly Of A Gene Coding For Human Proinsulin

The scheme used for assembling a gene coding for human proinsulin is shown in Fig. 3.

The synthetic gene segment coding for the first 31 amino acids of proinsulin, fragment 1 in Fig. 3, was recovered from 50 µg of the plasmid pIB3 using the restriction endonucleases EcoRI and HpaII as described above. This fragment

-16-

also contains the codon ATG for methionine in place of the "presequence" of preproinsulin. Introduction of a methionine unit at this point permits the polypeptide ultimately expressed to be cleaved at this point by cyanogen bromide (CNBr) to separate the proinsulin from the residue of the polypeptide which served to protect the proinsulin portion from bacterial proteases.

The cDNA gene segment coding for amino acids 32-86, as well as the translation stop codons and the 3' untranslated region of the mRNA was recovered from 40 µg of the plasmid pHI104 by treatment first with BamHI and then HpaII as shown in Fig. 3 as fragment 2.

The two fragments were isolated by polyacrylamide electrophoresis followed by electroelution. The gene fragments were joined by treatment with T4 DNA ligase in 20 µl ligase buffer (Goeddel et al. Proc. Nat. Acad. Sci., USA, 76, 106 (1979) at 4°C for 24 hrs. The mixture was diluted with 50 µl H₂O, extracted with phenol, then chloroform and then precipitated with ethanol.

The resulting DNA was treated with BamHI and EcoRI to regenerate these sites and remove gene polymers. The assembled proinsulin gene was isolated by polyacrylamide gel electrophoresis and ligated using T4 ligase to the plasmid pBR322 which had previously been treated with EcoRI and BamHI. The resulting DNA was transformed into E. coli K-12 strain 294 and cloned. Colonies were screened using the plasmid conferred antibiotic resistance markers. The desired clones were tetracycline-sensitive (Tc$^s$) and ampicillin-resistant (Ap$^r$). Plasmid pHI3 was isolated from one such colony and the proinsulin was characterized by nucleotide sequence analysis and found to have the sequence shown in Fig. 1.

4. Construction of a Plasmid Designed to Express a Chimeric Protein Containing the Human Proinsulin Peptide

Plasmid pBRH1, (R.I. Rodriguez, et al., Nucleic Acids Research 6, 3267-3287 (1979) expresses ampicillin resistance and contains the gene for tetracycline resistance but, there being no associated promoter, does not express that resistance. The plasmid is accordingly tetracycline sensitive. By introducing a promoter-operator system in the EcoRI site, the plasmid can be made tetracycline resistant.

Plasmid pGM1 carries the E. coli tryptophan operon containing the deletion LE1413 (G.F. Miozzari, et al., (1978) J. Bacteriology 1457-1466)) and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoter-operator system. The plasmid, 20 μg, was digested with the restriction enzyme PvuII which cleaves the plasmid at five sites. The gene fragments 2 were next combined with EcoRI linkers (consisting of a self complementary oligonucleotide 3 of the sequence: pCATGAATTCATG) providing an EcoRI cleavage site for a later cloning into a plasmid containing an EcoRI site. The 20 μg of DNA fragments 2 obtained from pGM1 were treated with 10 units of T$_4$ DNA ligase in the presence of 200 pico moles of the 5'-phosphorylated synthetic oligonucleotide pCATGAATTCATG and in 20 μl T$_4$ DNA ligase buffer (20mM tris, pH 7.6, 0.5 mM ATP, 10 mM MgCl$_2$, 5mM dithiothreitol) at 4°C overnight. The solution was then heated 10 minutes at 70°C to halt ligation. The linkers were cleaved by EcoRI digestion and the fragments, now with EcoRI ends were separated using 5 percent polyacrylamide gel electrophoresis (herein after "PAGE") and the three largest fragments isolated from the gel by first staining with ethidium bromide, locating the fragments with ultraviolet light, and cutting from the gel the portions of interest. Each gel fragment, with 300 microliters 0.1xTBE, was placed in a dialysis bag and subjected to electrophoresis at 100 v for one hour in 0.1xTBE buffer (TBE buffer contains: 10.8 gm tris base, 5.5 gm boric acid, 0.09 gm $Na_2EDTA$ in 1 liter $H_2O$). The aqueous solution was collected from the dialysis bag, phenol extracted, chloroform extracted and made 0.2 M NaCl, and the DNA removed in $H_2O$ after EtOH precipitation.

pBRH1 was digested with EcoRI and the enzyme removed by phenol extraction followed by chloroform extraction and recovered in water after ethanol precipitation. The resulting DNA molecule was, in separate reaction mixtures, combined with each of the three DNA fragments obtained above and ligated with $T_4$ DNA ligase as previously described. The DNA present in the reaction mixture was used to tranform competent E. coli K-12 strain 294, K. Backman et al., Proc Nat'l Acad Sci USA 73, 4174-4198 [1976]) (ATCC no. 31446) by standard techniques (V. Hershfield et al., Proc Nat'l Acad Sci USA 71, 3455-3459 [1974]) and the bacteria plated on LB plates containing 20 µg/ml ampicillin and 5 µg/ml tetracycline. Several tetracycline-resistant colonies were selected, plasmid DNA isolated and the presence of the desired fragment confirmed by restriction enzyme analysis. The resulting plasmid was designated pBRHtrp.

pBRH trp was digested wih EcoRI restriction enzyme and the resulting fragment isolated by PAGE and electroelution. EcoRI-digested plasmid pSOMΔ11 (K. Itakura et al., Science 198, 1056 (1977); G.B. patent publication no. 2 007 676 A) was combined with this fragment. The mixture was ligated with $T_4$ DNA ligase as previously described and the resulting DNA transformed into E. coli K-12 strain 294 as previously described. Transformant bacteria were selected on ampicillin-containing plates. Resulting ampicillin-resistant colonies were screened by colony hybridization (M. Gruenstein et al., Proc Nat'l Acad Sci USA 72, 3951-3965 [1975]) using as a probe the trp promoter-operator-containing fragment isolated from pBRHtrp, which had been radioactively labelled with $P^{32}$. Several colonies shown positive by colony hybridization were selected, plasmid DNA was isolated and the orientation of the inserted fragments determined by restriction analysis employing restriction enzymes BglII and BamHI in double digestion. E. coli 294 containing the plasmid designated pSOM7△2.

Plasmid pBR322 was HindIII digested and the protruding HindIII ends in turn digested with S1 nuclease. The S1 nuclease digestion involved treatment of 10 µg of HindIII-cleaved pBR322 in 30 µl S1 buffer (0.3 M NaCl, 1 mM $ZnCl_2$, 25 mM sodium acetate, pH 4.5) with 300 units S1 nuclease for 30 minutes at 15°C. The reaction was stopped by the addition of 1 µl of 30 X S1 nuclease stop solution (0.8M tris base, 50 mM EDTA). The mixture was phenol extracted, chloroform extracted and ethanol precipitated), then EcoRI digested as previously described and the large fragment 1' obtained by PAGE procedure followed by electroelution. The fragment obtained has a first EcoRI sticky end and a second, blunt end whose coding strand begins with the nucleotide thymidine.

16 µg Plasmid pSOM7△2 was diluted into 200 µl of buffer containing 20 mM Tris, pH 7.5, 5 mM $MgCl_2$, 0.02 percent NP40 detergent, 100 mM NaCl and treated with 0.5 units EcoRI. After 15 minutes at 37°C, the reaction mixture was phenol extracted, chloroform extracted and ethanol precipitated and subsequently digested with Bgl II. The larger resulting fragment 3' was isolated by the PAGE procedure followed by electroelution. This fragment contains the codons "LE'(p)" for the proximal end of the LE' polypeptide, i.e., those upstream from the Bgl II site. The fragment 3' was next ligated to the fragment 4'. Fragment 4' is prepared by successive digestion of pTrp24 (prepared upon EcoRI digest of pThα1 (Biochemistry 80, 6096 (1980)) followed by Klenow polymerase I reaction to blunt the EcoRI residues. Bgl II digestion creates a linear fragment which was recircularized by reaction with the LE' containing Bgl II sticky and blunt ends) with BglII and EcoRI, followed by PAGE and electroelution. The ligation was done in the presence of T₄ DNA ligase to form the plasmid pSOM7Δ2Δ4, which was transformed into E. coli strain 294, as previously described.

Plasmid pSOM7Δ2 was Bgl II digested and the Bgl II sticky ends resulting made double stranded with the Klenow polymerase I procedure using all four deoxynucleotide triphosphates. EcoRI cleavage of the resulting product followed by PAGE and electroelution of the small fragment 2' yielded a linear piece of DNA containing the tryptophan promoter-operator and codons of the LE' "proximal" sequence upstream from the Bgl II site ("LE'(p)"). The product had an EcoRI end and a blunt end resulting from filling in the Bgl II site. However, the Bgl II site is reconstituted by ligation of the blunt end of the fragment 2' to the blunt end of fragment 1'. Thus, the two fragments were ligated in the presence of T₄ DNA ligase to form the recircularized plasmid pHKY 10 which was propagated by transformation into competent E. coli strain 294 cells. Tetracycline resistant cells bearing the recombinant plasmid pHKY 10 were grown up, plasmid DNA extracted and digested in turn with Bgl II and Pst followed by isolation by the PAGE procedure and electroelution of the large fragment, a linear piece of DNA having Pst and Bgl II sticky ends to give DNA fragment 7'.

Plasmid pSOM7Δ2Δ4 could be manipulated to provide a second component for a system capable of receiving a wide variety of heterologous structural genes. The plasmid was subjected to partial EcoRI digestion followed by Pst digestion and the fragments containing the trp promoter/operator was isolated by the PAGE procedure followed by electroelution. Partial EcoRI digestion was necessary to obtain a fragment which was cleaved adjacent to the 5' end of the somatostatin gene but not cleaved at the EcoRI site present between the ampicillin resistance gene and the trp promoter operator. Ampicillin resistance lost by the Pst I cut in the $ap^R$ gene could be restored upon ligation with fragment 5'.

In a first demonstration the third component, a structural gene for somatostatin (6') was obtained and purified by PAGE and electroelution.

The three gene fragments 7', 5' and 6' could now be ligated together in proper orientation, to form the plasmid SOM7Δ1Δ4.

The complete human proinsulin gene, including the N-terminal codons that code for methionine, was recovered from the plasmid pHI3 by treatment with EcoRI and BamHI and purified by gel electrophoresis. This gene, fragment 3 in Fig. 3, was joined to two other DNA fragments with T4 ligase; these are identified as fragments 4 and 5 in Fig. 3.

Fragment 4' contains a promoter and a carrier protein gene derived from the plasmid pSOM7Δ1Δ4 by partial digestion with EcoRI and complete digestion with PstI. This fragment contains an E. coli tryptophan (trp) promoter-operator, nine codons from the trp leader peptide, 190 codons from the trp E gene and an EcoRI cleavage site introduced in place of the trp E termination codon. (This gene construction will be referred to as trp LE' below.) The tryptophan attenuator region including the last 5 codons of the trp leader peptide sequence and the first two thirds of the trp E gene are deleted in this construction.

The trp E gene (trp LE'), contained in Fragment 4', is modified to incorporate an EcoRI site in place of the termination codon of the trp E gene as shown to give the correct reading frame with the inserted gene fragment 3.

This fragment is bounded at the opposite end by a PstI site derived from the pBR322 and incorporates the first half of the β-lactamase gene. The fragment was recovered from 20 μg of plasmid pSOM7Δ1Δ4 by partial digestion with EcoRI followed by treatment with PstI. The promoter containing fragments were isolated by polyacrylamide gel electrophoresis.

Fragment 5' was obtained from plasmid pHKY10. This plasmid is a derivative of pBR322 and contains a tryptophan promoter-operator in place of the tetracycline promoter. The HindIII site of pBR322 has been converted to a BglII site. The plasmid, 20 μg, was treated wtih PstI and BglII and the large fragment, designated 5 in Fig. 3, purified by polyacrylamide gel electrophoresis.

The two fragments 4 and 5 were ligated together to reassemble the gene for β-lactamase via a PstI site and confer ampicillin resistance (Ap^r). The ends then present an EcoRI site and a BglII site for insertion of a gene. These two sites cannot be ligated together due to nonhybridization of the 3' protruding ends and can only be joined by incorporating a DNA fragment that possesses 3' ends complementary to the EcoRI and BglII ends. The proinsulin gene, fragment 3 containing EcoRI and BamHI ends, is such a molecule. Thus, the three fragments, 5 μg of 4, 1 μg of 3 and 1 μg of 5 were combined and treated with T4 DNA ligase at 4°C for 24 hours in ligase buffer. Upon circularization to close the plasmid, the tryptophan promoter-operator controls expression of a fusion (chimeric) protein of which proinsulin is a portion. Tetracycline resistance (Tc^r) is also conferred.

The DNA mixture from the ligation was transformed in E. coli, K-12 strain 294 by the procedure of Goeddel et al., Nature, 281, 541 (1979). Colonies were selected that would grow on both ampicillin and tetracycline. Of 3 colonies tested, 2 were found by SDS polyacrylamide gel electrophoresis (J.F. Maizel, Jr., Meth. Virol., 5, 180 (1971)) to express a protein of the molecular weight expected of the trp LE'- proinsulin fusion. One plasmid, pHI7 was completely characterized as to DNA sequence of the incorporated gene and restriction analysis of the vector pBR322.

5.  Proinsulin Isolation

The plasmid pHI7 was transformed into E. coli K-12 strain RV308 (ATCC No. 31608) and grown in 500 ml of LB medium (J.H. Miller, Experiments In Molecular Genetics, 433, Cold Spring Harbor 1972) containing 10 μg/ml of ampicillin to a cell density of 5 OD. This was diluted into a 10 liter fermentation vessel (New Brunswick) and grown in M9 media (Miller, supra, at 431.) to a cell density of 14 OD. Cells were collected by centrifugation and frozen.

Cells (164g) were thawed in 5 volumes sucrose lysis buffer (10 percent sucrose, 0.1M tris HCl, pH 7.9, 50mM EDTA, 0.2M NaCl) containing 0.1mM phenylmethylsulfonyl fluoride and 1.0 mM dimercaptopropanol, and lysed by sonication. The lysis pellet was collected by centrifugation and suspended by stirring overnight at 4°C with 4 volumes of 7.0M guanidine-HCl, 1mM dimercaptopropanol, 1mM EDTA. After centrifugation the supernatant was diluted 20 times with cold water and allowed to stand 2 hours at 4°C. The precipitate (9.6g dry) was collected by centrifugation and reacted overnight at room temperature in 220 ml 88 percent formic acid with 5g CNBr to cleave the proinsulin from the trp LE' fusion. After rotary evaporation the residue was suspended in 200 ml 7.5M urea, 1mM EDTA, 20mM ammonium carbonate, and the pH adjusted to 9.0 with ethanolamine (5.5 ml). Ten grams of sodium sulfite and five grams of sodium tetrathionate were added to convert cysteines and cystines to S-sulfonate groups and the reaction stirred at room temperature for 6 hours.

The reaction mixture was desalted on a G-25 Medium column in 7.5M urea, 10mM Tris pH 8.5, 10mM EDTA. The desalted protein was loaded onto a DEAE-sephadex (A-25) column and eluted with a 2-liter linear gradient of 0 to 0.5 M NaCl in the same tris-urea buffer. The proinsulin like material, identified by RIA or HPLC (see below), was concentrated on an Amicon YM5 membrane and resolved in the tris-urea buffer on a G-50 Medium column. The G-50 fractions, identified by HPLC, were pooled (104ml) and the buffer charged on a column of G-25 Fine equilibrated with 30 mM ammonium carbonate, pH 8.8. The lyophilized protein weighed 216 mg. The recoveries at each step are shown in Table 2.

6. Proinsulin Analysis

The S-sulfonated proinsulin obtained was analyzed by amino acid analysis. This analysis was made by Eli Lilly and Co. and is shown in Table 3.

TABLE 3

|  | Amino Acids Calculated | Amino Acids Predicted |  | Amino Acids Calculated | Amino Acids Predicted |
|---|---|---|---|---|---|
| Asp | 4.40 | 4 | Ile | 1.34 | 2 |
| Thr | 2.90 | 3 | Leu | 12.21 | 12 |
| Ser | 4.50 | 5 | Tyr | 3.93 | 4 |
| Glu | 15.64 | 15 | Phe | 2.61 | 3 |
| Pro | 3.42 | 3 | His | 2.02 | 2 |
| Gly | 11.08 | 11 | Lys | 1.96 | 2 |
| Ala | 4.46 | 4 | Arg | 3.92 | 4 |
| Cys | 2.85 | 6 | Val | 5.58 | 6 |

## TABLE 2

### PURIFICATION

| STEP | Wet Weight Material | Dry Weight Material | Protein (Lowry) | Proinsulin | Purification |
|---|---|---|---|---|---|
| Cells | 164 | -- | (21.2g) | 360mg (RIA) | -- |
| Sonication supernatant | -- | -- | 8.1g | -- | |
| Sonication pellet | 78 | -- | 13.1g | -- | 1.6 |
| Guanidine HCl supernatant | -- | -- | 11.5g | -- | |
| Guanidine HCl pellet | -- | -- | 0.3g | -- | |
| 20 x dilution pellet CNBr, sulfitolysis | -- | 9.6 | -- | -- | |
| G-25 Desalting Pool | -- | -- | 7.0g | 394mg (HPLC) | 2.0 |
| DEAE Pool | -- | -- | 776mg | 259mg (HPLC) | 6.0 |
| Amicon concentrate (YM5) | -- | -- | 369mg | 279mg (HPLC) | 2.2 |
| G-50 Pool | -- | -- | 390mg | 240mg (HPLC) | 0.8 |
| G-25 Pool | -- | -- | 209mg | 233mg (HPLC) | 1.6 |

The presence of proinsulin was also confirmed by radioimmunoassay. To determine the radioimmunoactivity the Corning $^{125}I$-insulin kit was used. The antibody was found to be about 4 percent cross-reactive with proinsulin and about 0.2 percent cross-reactive with reduced proinsulin. Unknowns were heated 2 minutes at 90°C, in 7.5M urea, 2 mM ß-mercaptoethanol, pH 8-10 (ethanolamine), and aliquots diluted in phosphate-buffered saline (0.1 gelatin) and immediately assayed. These results were determined from comparisons to a reduced proinsulin standard curve generated in the same way from either bovine proinsulin or human proinsulin S-sulfonate.

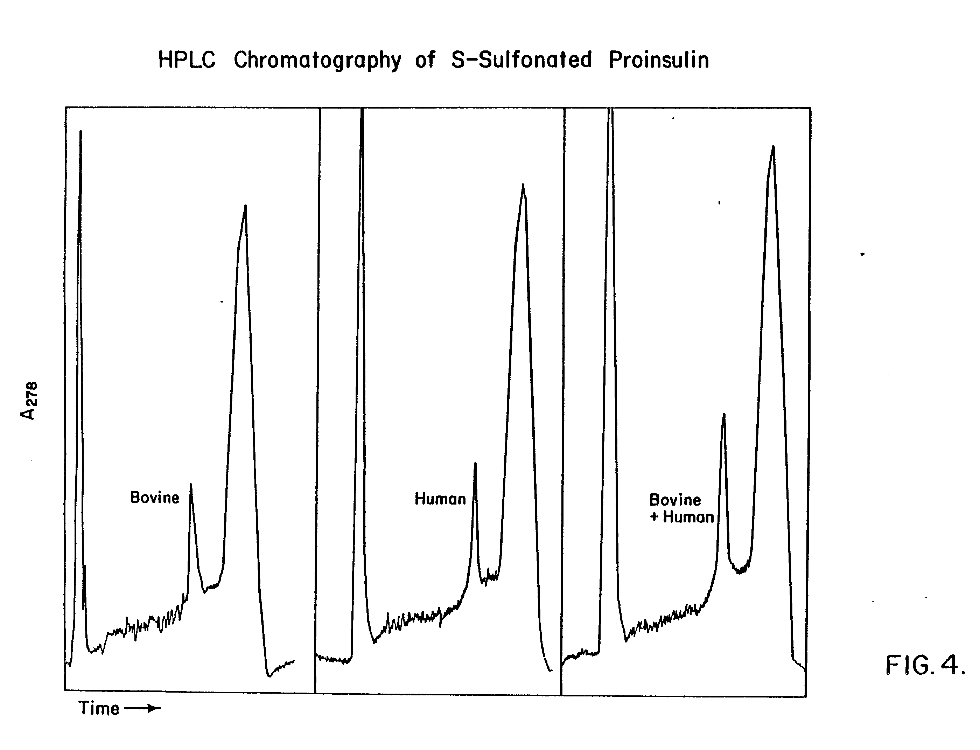

The proinsulin-S-sulfonate was also assayed by HPLC. In Fig. 4 profiles are shown for S-sulfonated Bovine proinsulin, bacterial derived human proinsulin and a combination of the two. In this analysis, samples of bovine, and human proinsulin sulfonate and a mixture of the two were applied to a 10 m RP-189 column and eluted using a linear gradient of 21 to 33 percent n-propanol and acetonitrile (2:1) in 50 mM $_4$NH OAc (pH7). The proteins are seen to run very nearly coincident. The large $A_{278}$ peak at the end of the chromatogram is due to rapid changes in the solvent composition and not eluted protein.

B. Preparation of Proinsulin Analog

Described below is the synthesis of a gene which codes for the expression of an analog of proinsulin comprising the A and B chains of human insulin connected by a bridging chain which differs from the C chain of human proinsulin in that it contains only 6 amino acid units rather than the 35 unit polypeptide of human proinsulin shown in Fig.1. Specifically the 6 units are, reading in order from the last unit of the B chain to the A chain, Arg-Arg-Gly-Ser-Lys-Arg. This sequence has the same end sequences Arg-Arg and Lys-Arg as does human

proinsulin thus permitting excision of the bridging chain by proteolytic means.

A chain of 6 amino acids is an acceptable length for a modified (or analog) bridging "C" chain which will permit folding and the subsequent formation of the disulfide crosslinks between A and B chains characteristic of hormone insulin. However, those skilled in the art will appreciate that bridging chains shorter or longer than 6 would also be useful as well by permitting folding and the formation of the necessary disulfide bonds. Sequences of 100 or even more amino acid units can be employed in the bridging chain. However, the practical difficulty of obtaining gene fragments coding for very long sequences makes the bridging chain analogs of fewer than 35 amino acid units more attractive from a practical point of view.

The ends of the bridging chain, no matter how many intermediate amino acid units or in what order, must be constructed to permit excission of the bridging chain. Although alternative means may be employed, we prefer to use the sequences Arg-Arg and Arg-Lys as found in proinsulin itself as proteolytic cleavage using trypsin and carboxypeptidase B occurs cleanly at these sites.

1. Preparation of Synthetic Gene Coding for the 57 Amino Acids of an Analog Proinsulin

    a. Oligonucleotide Synthesis

        The chemical synthesis methods as well as the synthesis of the DNA gene fragments coding for the A and B chains of human insulin have been described. K. Itakura et al., J. Biol. Chem., 250, 4592 (1975), K. Itakura et al. J. Biol. Chem., 250, 4592 (1975), K. Itakura et al. J. Am. Chem. Soc., 97, 7327 (1975), Crea et al. Proc. Nat. Acad. Sci., USA, 75, 5765 (1978) and Goeddel et al. Proc. Nat. Acad. Sci., USA,

-28-

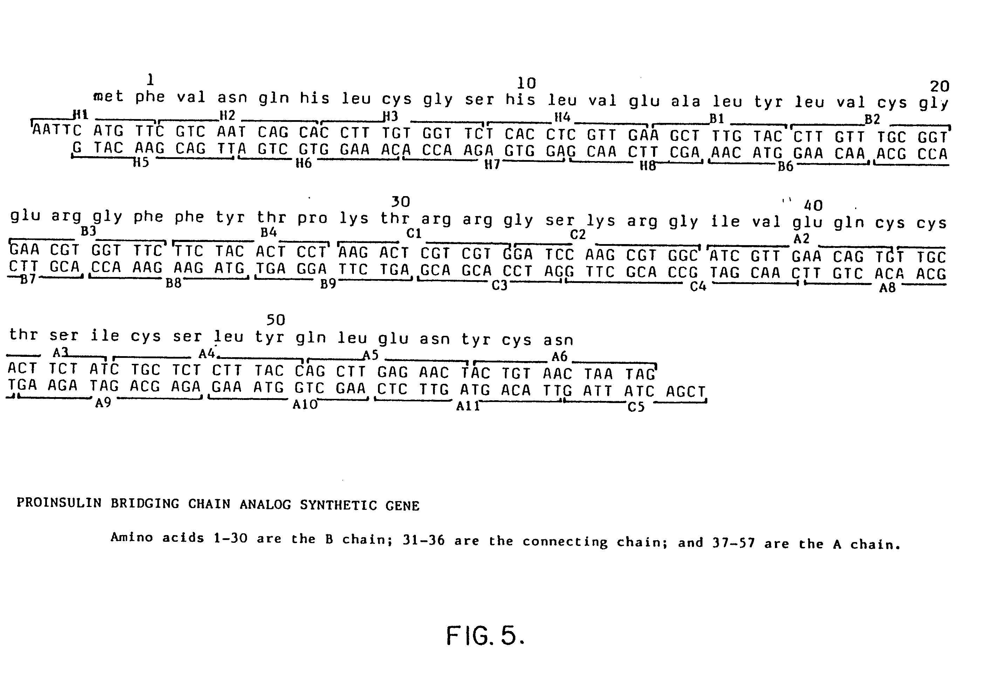

<u>76</u>, 106 (1979). Five new oligonucleotide fragments were synthesized by similar methods also using the triester process described in the above cited references. These sequences are shown below in Table 4 and Fig. 5.

<u>TABLE 4</u>

<u>Oligonucleotides</u>

| $C_1$ | AAGACTCGTCGTG |
|-------|---------------|
| $C_2$ | GATCCAAGCGTGGCATC |
| $C_3$ | GATCCACGACGAGTCTT |
| $C_4$ | CAACGATGCCACGCTTG |
| $C_5$ | TCGACTATTAGTT |

b. Joining of Synthetic Oligonucleotides

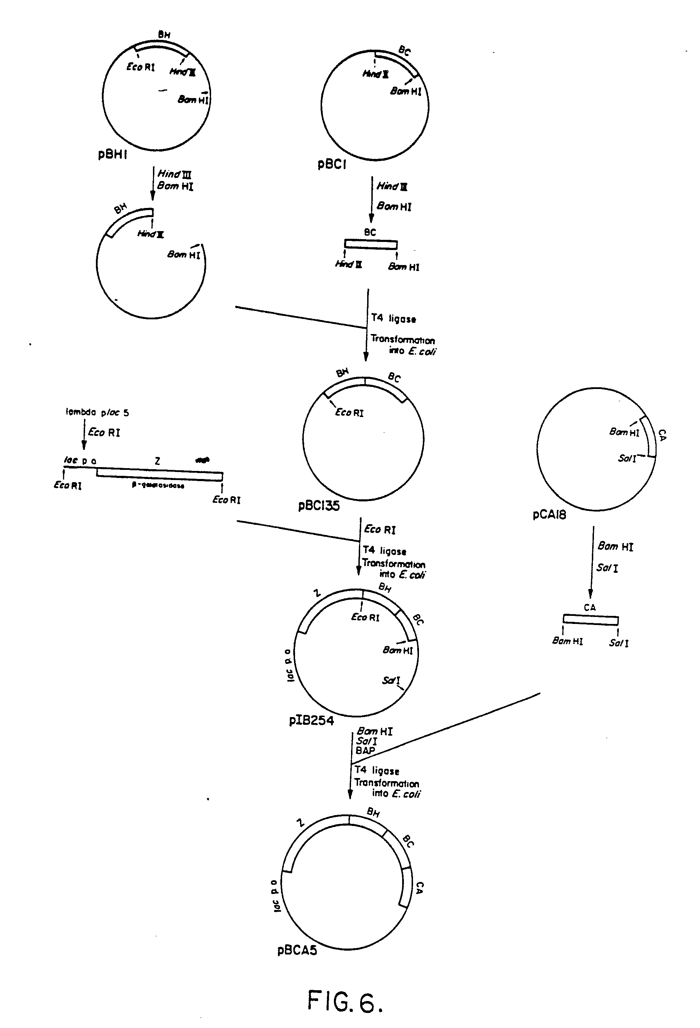

Figure 5 shows the synthetic oligonucleotides of the insulin A and B chain genes previously prepared and the manner in which the new fragments $C_1$-$C_5$ were used in the enzymatic construction of a complete gene for a proinsulin analog. The scheme for obtaining this gene is set forth in Fig. 6.

A plasmid pBH1 containing the left half of the B chain gene was used in this process and is described by Goeddel et al., <u>Proc. Nat. Acad. Sci., USA</u>, <u>76</u>, 106 (1979). It contains the codons for the 1-13 amino acids of the B chain and a methionine unit at the N-terminal which will be used later to cleave the proinsulin analog from the bacterially expressed chimeric protein using cyanogen bromide (CNBr).

The gene for the right half of the B chain was obtained from the oligonucleotides $B_1$, $B_2$, $B_3$, $B_4$, $C_1$, $B_6$ and $B_7$, $B_8$, $B_9$ and $C_3$ ($C_1$ and $C_3$ replacing the

$B_5$ and $B_{10}$ sequences in the previously prepared gene fragment) by ligation using T4 ligase in conventional techniques. This gene fragment codes for the 14-30 amino acid units of the B chain and the first two units Arg-Arg of the bridging (modified "C") chain-pBC1.

After purification by polyacrylamide gel electrophoresis and elution of the largest DNA band the fragment was inserted into the plasmid pBR322 that had been cleaved with restriction endonucleases HindIII and BamHI, thereby utilizing the HindIII and BamHI sites on the gene fragment, and cloned in E. coli 294 (ATCC No. 31446). The plasmid pBC recovered from an ampicillin-resistant, tetracycline sensitive clone possessed the desired nucleotide sequence according to the method of A.M. Maxam et al., Proc. Nat. Acad. Sci., USA, 74, 560 (1977).

The A gene was constructed similarly from oligonucleotides $C_2$, $A_2$, $A_3$, $A_4$, $A_5$, $A_6$, $C_4$, $A_8$, $A_9$, $A_{10}$, $A_{11}$ and $C_5$ ($C_2$, $C_4$ and $C_5$ replacing the $A_1$, $A_2$ and $A_{12}$ sequences in the previously prepared gene fragment) using T4 ligase in conventional techniques. This fragment codes for the 21 amino acids of the A and the four units of the bridging C chain, Gly-Ser-Lys-Arg. After purification, also by polyacrylamide electrophoresis, the fragment was inserted into the plasmid pBR322 which had been cleaved with restriction endonucleases EcoRI and SalI using the EcoRI and SalI sites on the fragment and cloned in E. coli 294. An ampicillin-resistant, tetracycline-sensitive clone yielded plasmid pCA18 having the desired nucleotide sequence by the method of A.W. Maxam et al., supra.

2. Construction of the Proinsulin Analog Gene and Corresponding Expression Plasmid

The desired expression plasmid was prepared from plasmids pBH1, pBC1 and pCA18 as shown in Fig. 6. The plasmid pBH1 was cleaved with <u>Hind</u>III and ligated to the fragment BC
excised from plasmid pBC1 by treatment with <u>Hind</u>III and <u>Bam</u>HI.
The resulting plasmid pBC135 was cleaved with <u>Eco</u>RI and ligated
to an <u>Eco</u>RI fragment of p<u>lac</u>5 which contains the <u>lac</u> control
region and the majority of the ß-galactosidase structural gene
(designated Z). K. Itakura <u>et al</u>. <u>Science</u>, <u>198</u>, 1056 (1977).
This ligation produced plasmid pIB254 which was cleaved with
<u>Bam</u>HI, <u>Sal</u>I and alkaline phosphatase. The product of this
cleavage was ligated to fragment CA excised from plasmid pCA18
by treatment with <u>Bam</u>HI and <u>Sal</u>I as shown in Fig. 6 and trans-
formed into <u>E</u>. <u>coli</u> 294 and cloned. The plasmid pBCA5 was
recovered from ampicillin-resistant, tetracycline-sensitive
clones in <u>E</u>. <u>coli</u> 294 grown on X-gal plates containing
ampicillin and contained the DNA coding sequence for the
proinsulin analog as indicated by the method of A.M. Maxam
<u>et al</u>., <u>supra</u>.

3. Expression of Proinsulin Analog

The fully characterized plasmid pBCA5 was inserted into
<u>E</u>. <u>coli</u> RV308 and grown in four-liter flasks containing 1.5
liter LB containing 20 mg/l ampicillin. Recovered cells (322 g
wet) were lysed by sonication in two liters of 10 percent
sucrose, 50 mM EDTA, 0.1 M tris/HCl, pH 7.9, 0.1 N
phenylmethylsulfonylfluoride, 0.2 M NaCl, and 1 mM
1,3-dithio-2-propanol. After centrifugation (30 min, 5000 rpm)
the pellet was suspended by stirring in 400 ml 7M guanidine
hydrochloride, 0.1 mM, 1,3-dithio-2-propanol, 1 mM EDTA. This
suspension was centrifuged (30 min, 12,000 rpm) and the
supernatant diluted six-fold into cold water. The
precipitation protein (12.4 g dry weight) was collected by
centrifugation (20 min, 5000 rpm) and treated overnight at RT
with 2.8 g (26.4 mmol) CNBr in 200 ml 88 percent formic acid to
cleave the proinsulin analog (hereinafter "analog-'C'"

proinsulin) from the ß-galactosidase residues at the
N-methionine unit. After rotary evaporation to dryness at
under 30°C, water was added and the volume again reduced. The
residue was suspended in 300 ml 6 M guanidine hydrochloride and
the pH adjusted to 9.0 with ethanolamine. To this was added
15.0 g sodium sulfite and 7.5 g sodium tetrathionate to convert
cysteines and cystines to cysteine S-sulfonate groups and the
mixture allowed to react at room temperature for six hours.
The reaction mixture was exhaustively dialyzed (Spectropor 3)
against 1 mM EDTA at 4°C and the precipitated protein
S-sulfonates collected by centrifugation (20 min, 5000 rpm).
The pellet was suspended in 50 ml 7.5 mM Tris/HCl, pH 7.5,
filtered and loaded onto a DE-52 column (2.5 x 87 cm) in the
same buffer at 4°C. The column was eluted with a linear
gradient of 0 to 0.5 M NaCl in the same buffer. The presence
of mini-C proinsulin S-sulfonate was detected by insulin RIA
analyses described below to elute at the end of the OD peak.
The pooled fractions were applied to a G-50F Sephadex column
(2.5 x 100 cm) and eluted with 7 M urea, 50 mM tris/HCl, pH
7.5, 1 mM EDTA. Fractions containing insulin-RIA-active
material were pooled and dialyzed against 20 mM ammonium
carbonate, pH 8.8, and lyophilized. The white powder was
resuspended in 7 ml 20 mM ammonium carbonate, pH 8.8, and
stored at -40°C. A portion of the product pool was further
purified by preparative HPLC and the insulin RIA-active peak
analyzed for amino-acid content which is shown in Table 5.

The analog-"C" proinsulin S-sulfonate was purified by
HPLC by prep-collection from an analytical C-8 column. The
resolving gradient was 21-39 percent 2-propanol in 50 mM NaOAc,
pH 7.0, at 0.6 percent/min and 2 ml/min. Up to 1.5 mol. of
protein solution was resolved in one run by three successive
injections via a 500 µl loop.

## TABLE 5

Amino Acid Analysis of 24-hour 6N HCl hydrolysis (110°) of purified analog-"C" proinsulin. Cysteine was quantitated by separate determination of cysteic acid on performic acid oxidized sample and calculated by cysteic/alanine ratio. Values increased to compensate for acid decomposition were serine (10 percent) and threonine (5 percent). N.D. = not determined.

| Amino Acid predicted | aa mole percent x 57 | amino acids |
|---|---|---|
| Ala | 1.19 | 1 |
| Arg | 4.06 | 4 |
| Asp | 3.17 | 3 |
| Cys/2 | 4.87 | 6 |
| Glu | 6.95 | 7 |
| Gly | 5.37 | 5 |
| His | 2.03 | 2 |
| Ile | 1.39 | 2 |
| Leu | 5.98 | 6 |
| Lys | 2.02 | 2 |
| Met | 0 | 0 |
| Phe | 3.03 | 3 |
| Pro | 1.96 | 1 |
| Ser | 4.21 | 4 |
| Thr | 3.09 | 3 |
| Trp | N.D. | 0 |
| Tyr | 3.99 | 4 |
| Val | 3.74 | 4 |

4. Folding Of Analog "C" Proinsulin

Analog "C" proinsulin folding to obtain a crosslinked form was accomplished by reaction of 4 mg of protein containing about 30 percent analog "C" proinsulin sulfonate. The protein was dissolved in a degassed buffer of 40 mM glycine, pH 10.6, 3 M urea, 0.3 M NaCl at 0°. To this was added ß-mercaptoethanol to a concentration of 0.4 mM and the reaction sealed under $N_2$. The course of the reaction was followed by measuring the increase in RIA activity and was complete within about four hours. The reaction was stopped by the addition of acetic acid.

The reaction mixture was purified by HPLC by prep-collection from a C-18 Ultrosphere column. The resolving gradient was 21-28 percent acetonitrile in 0.2m ammonium sulfate, 50 mM NaOAc, pH 4.0, at 0.2 percent/min and 1.0 ml/min.

5. Assay For Analog "C" Proinsulin

Analog "C" proinsulin has no crossreactivity in the insulin radioimmuno assay as the S-sulfonate. However, the formation of analog "C" proinsulin as an expression product was confirmed by crossreactivity of the thiol form. The samples of unknowns were treated for two minutes at 90°C with 1 mM ß-mercaptoethanol, diluted into RIA buffer (0.1 M sodium phosphate, pH 7.4, 0.15M NaCl, 0.1 percent $NaN_3$, 0.1 percent gelatin) and immediately assayed. Reproduceability depends upon strict timing since extended incubation of the diluted, reduced test solution leads to variable oxidative folding of the molecule into forms with higher RIA activity.

The thiol form gave an activity of 0.9 percent compared to insulin's 100 percent. By comparison, bovine proinsulin had RIA activity of 7.3 percent of insulin activity and the thiol form of bovine proinsulin had an activity of 1.9 percent. The third form of the B chain of porcine proinsulin had activity of 0.1 percent. On incubation with a slight excess of

ß-mercaptoethanol to obtain the folded, crosslinked product, reaction mixtures show RIA activity of 20-40 percent that of insulin.

C.  Preparation of Human Insulin

1.  Folding and Linking of A and B Chains.

The human proinsulin or analogs thereof, prepared in accordance with the present invention, for example, following the procedures of Parts A or B above, as their respective S-sulfonates are induced to fold with proper formation of internal disulfide bonds (between cysteine $_7$B and cysteine $_7$A, between cysteine$_{19}$ B and cysteine$_{20}$ A, and between· cysteine$_{6,11}$ A) by means of controlled sulfhydroxyl interchange catalyzed by ß-mercaptoethanol.

To a 0.1 mg/ml solution of proinsulin S-sulfonate in degassed 50 mM sodium glycinate, pH 10.6, at 4°C was added· ß-mercaptoethanol to a final concentration of 0.3 mM.  After four hours, the reaction is essentially complete as measured by the increase in cross-reacting activity of the mixture in insulin RIA.  The yield of proinsulin is about 80 percent. Proinsulin is then purified from side products by gel permeation, ion exchange, and/or reverse phase high pressure liquid chromatography to yield product in substantially purified form.

2.  Excision of Bridging Chain

The human proinsulin or analogs thereof, prepared in accordance with the present invention, for example, following the procedure of Part C. 1. above, are proteolytically converted to insulin for example in accordance with the procedure of Kemmler et al., J. Biol Chem., 246, 6786 (1971).  The obtained insulin is then purified by column chromatography or zinc crystallization to yield product in substantially form, identical to natural human insulin and freed of biologically active contaminants.

While the invention in its most preferred embodiment is described with reference to E. coli, other microrganisms could likewise serve as host cells, for example, yeasts such as Saccharomyces cerevisiae, Bacilli such as Bacillus subtilis and preferably other enterobacteriaceae among which may be mentioned as examples Salmonella typhimurium and Serratia marcesans, utilizing plasmids that can replicate and express heterologous gene sequences in these organisms. The invention is not to be limited to the preferred embodiments described.

CLAIMS:

1.    A chimeric polypeptide comprising:

      a) the polypeptide sequence of a proinsulin comprising the A and B chains of human insulin connected by a bridging chain of at least 2 amino acid units, said bridging chain having sites at each end which permit its excision from between said A and B chains; and

      b) an additional protein or protein fragment; there being a cleavage site at or adjacent said additional protein or fragment and adjacent one end of the polypeptide sequence of said proinsulin.

2.    A chimeric polypeptide according to claim 1 wherein the amino acid sequence of the bridging chain corresponds to that of the C peptide of human proinsulin.

3.    A chimeric polypeptide according to claim 1 wherein the amino acid sequence of the bridging chain is Arg-Arg-Gly-Ser-Lys-Arg.

4.    A chimeric polypeptide according to claim 1 wherein the amino acid sequence of the bridging chain is Arg-Arg.

5.    A chimeric polypeptide according to claim 1 or claim 2 wherein the sites permitting excision of the bridging chain are the amino acid units Arg-Arg at the  B chain end and Lys-Arg at the A chain end.

6.   A chimeric polypeptide according to any one of claims 1 to 5 wherein said cleavage site is a methionine unit.

7.   A chimeric polypeptide according to claim 6 wherein the methionine unit is adjacent the N-terminal of said proinsulin.

3.   A chimeric polypeptide according to any one of claims 1 to 7 wherein the additional protein or fragment is either

       a) at least a substantial portion of β-galactosidase; or

       b) a fragment of the trp leader polypeptide fused to a portion of the trp E polypeptide.

9.   A process of producing a chimeric polypeptide according to claim 1 comprising the steps:

       1) inserting a gene coding for said proinsulin into a microbial cloning vehicle in which the gene is in reading phase with a DNA sequence coding for said additional protein or fragment comprising said cleavage site;

       2) transforming said cloning vehicle containing said inserted gene into a microbial host for expression of said chimeric polypeptide;

       3) expressing the chimeric polypeptide; and

       4) isolating the expressed chimeric polypeptide.

10.   A process for producing a proinsulin comprising the process of claim 9 and the additional step of cleaving said chimeric polypeptide to release said proinsulin.

11.   A process for producing a protein comprising the process of claim 10 and the additional step of excising said bridging chain from said proinsulin.

12.   A process according to claim 11 wherein the excision of the bridging chain is preceded by the formation of disulfide bonds between the A and B chains and the product of excision is human insulin.

13.   Human proinsulin when prepared by the process of claim 10.

14.   Human insulin when prepared by the process of claim 12.

15.   A cloning vehicle suited for transformation of a microbial host and use therein for expressing a chimeric polypeptide according to claim 1.

16.   The plasmid pH17.

17.   The plasmid pBCA5.

18.   A viable culture of microbial transformants containing a cloning vehicle according to any one of claims 15 to 17.

19.   A method of producing human insulin comprising: 1) cultivating a culture according to claim 18; 2) separating the resulting cellular mass; 3) isolating the precursors to human insulin comprising the chimeric polypeptide according to claim 1; 4) cleaving the additional protein therefrom; 5) effecting folding and linkage of the A and B chains; and 6) excising the bridging chain.

20.    A product of microbial expression comprising the polypeptide sequence of a proinsulin comprising the A and B chains of human insulin connected by a bridging chain of at least 2 amino acid units and from which human insulin is derivable upon excision of said bridging chain.

```
              1                              10                                    20
   met phe val asn gln his leu cys gly ser his leu val glu ala leu tyr leu val cys gly
AATTC ATG TTC GTC AAT CAG CAC CTT TGT GGT TCT CAC CTT GTT GAA GCT CTC GTT TAC CTT GTT TGC GGT
  G TAC AAG CAG TTA GTC GTG GAA ACA CCA AGA GTG GAA CAA CTT CGA GAG CAA ATG GAA CAA ACG CCA
   H1        H2        H3         H4         H5        H7        H8        B1        B2

                                                   30                        40
glu arg gly phe phe tyr thr pro lys thr arg arg glu ala glu asp leu gln val gly gln val glu
GAA CGT GGT TTC TTC TAC ACT CCT AAG ACT CGC CGG GAG GCA GAG GAC CTG CAG GTG GGG CAG GTG GAG
CTT GCA CCA AAG AAG ATG TGA GGA TTC TGA GCG GCC CTC CGT CTC CTG GAC GTC CAC CCC GTC CAC CTC
   B3         B4       B5       B5'   B10

                                    50
leu gly gly pro gly ala gly ser leu gln pro leu ala leu glu gly ser leu gln lys arg gly
CTG GGC GGC CCT GGT GCA GGC AGC CTG CAG CCC TTG GCC CTG GAG TCC CTG CAG AAG CGT GGC
GAC CCG CCG GGA CCA CGT CCG TCG GAC GTC GGG AAC CGG GAC CTC AGG GAC GTC TTC GCA CCG

              70                           80
ile val glu gln cys cys thr ser ile cys ser leu tyr gln leu glu asn tyr cys asn
ATT GTG GAA CAA TGC TGT ACC AGC ATC TGC TCC CTC TAC CAG CTG GAG AAC TAC TGC AAC TAG ACCAGC
TAA CAC CTT GTT ACG ACA TGG TCG TAG ACG AGG GAG ATG GTC GAC CTC TTG ATG ACG TTG ATC TGGGTCG

CCGCAGGCAGCCCCCACCCCGCCCTCCTGCACCGAGAGAGATGGAATAAAGCCCTTGAACCAGC-polyA-CCG
GGCGTCCGTCGGGGGTGTGGGCGGGAGGACGTGGCTCTCTCTACCTTATTTCGGGAACTTGGTCG-polyT-GGCCTAG
```

PROINSULIN SYNTHETIC GENE

Amino acids 1-30 are the B chain; 31-65 are the C chain; and 66-86 are the A chain.

FIG.I.

FIG. 2.

pIB3

Eco RI        Hpa II   BamHI

1   2
phe phe met phe val
GAA TTC ATG TTC GTC
CTT AAG TAC AAG CAG

29 30 31 32
lys thr arg arg
AAG ACT CGC CGG ATG C
TTC TGA GCG GCC TAGG

pHI104

Pst I      Hpa II    Pst I     BamHI   Pst I

GGGGGG ─────────── CCCCCC
CCCCCC ─────────── GGGGGG

29 30 31 32 33
lys thr arg arg glu
AAG ACC CGC CGG GAC
TTC TGG GCG GCC CTG

AAAACCGGATCCGGCCCC
TTTTGGCCTAGGCCGGGG

Eco RI , Hpa II           BamHI, Hpa II

Eco RI       Hpa II       Hpa II       BamHI

**1**              **2**

pSOMΔIΔ4

Eco RI partial, Pst I

T4 ligase
Eco RI , BamHI

Pst I    Eco RI   trp p.o    Δ    LE' fusion    Eco RI

Ap^R

**4**

L8 L9 E339 E340
lys gly ser leu
AAA GGT TCG CTG
TTT CCA AGC GAC

E516 E517
phe phe phe phe
GCA CAG G
CGT GTC CTT AA

Eco RI                BamHI

**3**

Pst I           Bgl II

Ap^R    pHKY_{10}    Tc^R
Δ Pst I - Bgl II

ori

**5**

T4 ligase
Transformation

trp p.o   LE' fusion   proinsulin

Ap^R    pHI7

Tc^R

ori

FIG. 3.

0055945

# HPLC Chromatography of S-Sulfonated Proinsulin

A_278

Bovine

Human

Bovine
+ Human

Time ⟶

FIG. 4.

9/4

0055945

```
      1                                          10                                        20
    met phe val asn gln his leu cys gly ser his leu val glu ala leu tyr leu val cys gly
 ┌──H1──────┬───────H2───────────┬──────H3──────────┬──────H4───────┬──────B1──────┬─────B2──────┐
 AATTC ATG TTC GTC AAT CAG CAC CTT TGT GGT TCT CAC CTC GTT GAA GCT TTG TAC CTT GTT TGC GGT
 G TAC AAG CAG TTA GTC GTG GAA ACA CCA AGA GTG GAG CAA CTT CGA AAC ATG GAA CAA ACG CCA
 └─────H5──────┴──────H6──────┴──────H7──────┴──────H8──────┴─────B6──────┘

 glu arg gly phe phe tyr thr pro lys thr arg arg gly ser lys arg gly ile val glu gln cys cys
 ┌────B3──────┬──────B4──────┬──────C1──────┬──────C2──────┬──────────┬─────A2──────┐
 GAA CGT GGT TTC TTC TAC ACT CCT AAG ACT CGT CGT GGA TCC AAG CGT GGC ATC GTT GAA CAG TGT TGC
 CTT GCA CCA AAG AAG ATG TGA GGA TTC TGA GCA GCA CCT AGG TTC GCA CCG TAG CAA CTT GTC ACA ACG
 └─B7──┴──────B8──────┴──────B9──────┴──────C3──────┴──────C4──────┴──────A8──────┘

                  50
 thr ser ile cys ser leu tyr gln leu glu asn tyr cys asn
 ┌───A3───┬──────A4──────┬──────A5──────┬──────A6──────┐
 ACT TCT ATC TGC TCT CTT TAC CAG CTT GAG AAC TAC TGT AAC TAA TAG
 TGA AGA TAG ACG AGA GAA ATG GTC GAA CTC TTG ATG ACA TTG ATT ATC AGCT
 └─────A9──────┴──────A10──────┴──────A11──────┴──────C5──────┘
```

PROINSULIN BRIDGING CHAIN ANALOG SYNTHETIC GENE

Amino acids 1-30 are the B chain; 31-36 are the connecting chain; and 37-57 are the A chain.

FIG. 5.

FIG.6.